Europäisches Patentamt

**European Patent Office**

**Office européen des brevets**

(11) Publication number: **0 015 030**
A2

(12) # EUROPEAN PATENT APPLICATION

(21) Application number: **80200123.0**

(22) Date of filing: **14.02.80**

(51) Int. Cl.³: **A 61 K 7/48**

(30) Priority: **23.02.79 US 14459**

(43) Date of publication of application:
**03.09.80 Bulletin 80/18**

(84) Designated Contracting States:
**AT BE CH DE FR GB IT LU NL SE**

(71) Applicant: **THE PROCTER & GAMBLE COMPANY**
**301 East Sixth Street**
**Cincinnati Ohio 45202(US)**

(72) Inventor: **Tollet, Carol Norris**
**3445 Wellston Place**
**Cincinnati, Ohio 45208(US)**

(74) Representative: **Gibson, Tony Nicholas et al,**
**Procter & Gamble (NTC) Limited Whitley Road**
**Longbenton Newcastle upon Tyne NE12 9TS(GB)**

(54) Skin conditioning compositions.

(57) Skin conditioning compositions, especially compositions for the care of the face, comprising an emulsion which is about 8 to 25% of skin moisturizing ingredients of which about 2.5 to 8% is a sterol, about 10 to 35% is a sugar or a hydroxy alcohol and 5 to 15% is lanolin fatty acids. An emulsifier is also present as a skin moisturizing ingredient. Other oily, moisturizing materials may make up the remainder of the moisturizing fraction.

EP 0 015 030 A2

Croydon Printing Company Ltd.

SKIN CONDITIONING COMPOSITIONS

The present invention is related to skin conditioning compositions which possess a unique combination of moisturizing materials. The moisturizing combination provides excellent skin conditioning benefits while also being very easy to rub in (i.e. very spreadable and quick to rub in) and not leaving an undesirable greasy residue on the skin.

The treatment of human skin with various agents has been undertaken for many years with the goal being to keep the skin in a smooth and supple condition. Skin has the tendency to dry out when extended periods of exposure to the elements and cleaning solutions are encountered. From a biochemical standpoint, dryness is a measure of the water content of the skin. Under normal conditions, the water content and vapor pressure of the epidermis are higher than those of the surrounding air with consequent evaporation of water from the skin surface. Skin becomes dry because of excessive loss of water from the surface and the subsequent loss of water from the stratum corneum.

The facial areas present special problems in addition to those present with, for instance, the hands. Some facial areas, due to high concentrations of sebaceous and sweat glands, often feel greasy to a person. Other facial areas do not feel this way and may feel overly dry.

The facial areas are also subject to wrinkling which causes difficulty in achieving good distribution of facial compositions. All of these problems require that special care be taken in formulating a product which is to be used on the face as well as other body areas.

## Background Art

To alleviate the aforementioned conditions, emollient creams as described in Sagarin, Cosmetics Science and Technology, 2nd Edition, Vol. 1, Wiley Interscience (1972) have been recommended for application to the skin. The emollient materials probably increase the state of hydration of the corneous layer of the skin by altering the rate of diffusion of water from the lower epidermal and dermal layers, the rate of evaporation of water from the skin's surface, and the ability of the corneous layer to hold moisture.

The use of three of the essential moisturizing components of the present compositions, lanolin fatty acids, sterols and a hydroxy alcohol or a sugar, has been disclosed in the art but not in the critical amounts found to be essential in the present invention. U.S. Patent 3,957,971, May 18, 1976 to Oleniacz discloses liposomes comprising lecithin, dicetyl phosphate, a sterol and an aqueous solution of a humectant. U.S. Patent 3,818,105, June 18, 1974 to Coppersmith et al discloses compositions containing isoparaffinic hydrocarbons and other cosmetic additives such as lanolin and a humectant such as propylene glycol. U.S. Patent Re. 29,814, October 24, 1978 to W. E. Snyder discloses compositions containing lanolin fatty acids, silicone, lanolin derivatives and sorbitol. Cholesterol in a variety of compositions is disclosed in Drug and Cosmetic Industry, Vol. 74, pp. 52, 53, 127 - 132 (1954).

## Disclosure of the Invention

The present invention relates to superior skin conditioning compositions comprising an emulsion which contains from about 8 to about 25%, preferably from about 12 to about 15%, of skin moisturizing materials of which about 2.5 to about 8% is a sterol, about 10 to 35% is a sugar, a penitol or a hexitol, and about 5 to about 15% is lanolin fatty acids. An emulsifier is also present as a skin moisturizing ingredient.

It has been discovered that the above-described components provide skin conditioning lotions which are easily rubbed into the skin and do not leave a greasy, undesirable residue.

## Detailed Description of the Invention

As discussed above, the present compositions comprise a moisturizing fraction having certain critical components. In addition, the compositions may contain additional oil soluble, skin conditioning materials to work in cooperation with the critical moisturizing components. Furthermore, the present emulsion compositions may also contain additional water soluble materials to improve the overall acceptability of the compositions. Each of the various components is discussed in detail below.

### Sterol

The sterol component used in the present compositions may be selected from any of the available animal or plant sterols. Preferably, the sterol is selected from the group consisting of cholesterol, phytosterol, sitosterol, sitosterol pyroglutomate, 7-dehydrocholesterol, lanosterol and mixtures thereof. The preferred sterol is cholesterol. The sterol is present at a level of from about 2.5 to about 8%, preferably from about 4 to 8%, by weight of the skin moisturizing fraction.

## Sugar or Hydroxy Alcohol

The sugar or hydroxy alcohol useful in the present compositions is selected from the group consisting of $C_5$ or $C_6$ monosaccharides, $C_5$ or $C_6$ disaccharides, pentahydroxy alcohols, hexahydroxy alcohols and mixtures thereof. Examples of suitable monosaccharides include arabinoses, xyloses, ribose, glucose, galactose, mannose and fructose. Suitable disaccharides include lactose, maltose, gentiobiose, cellobiose and sucrose. Suitable hydroxy alcohols include arabitol, xylitol, adonitol mannitol, sorbitol and dulcitol. The sugar or hydroxy alcohol is present at a level of from about 10 to 35%, preferably from about 10 to 25%, by weight of the moisturizing fraction. Sorbitol is the preferred sugar or hydroxy alcohol.

## Lanolin Fatty Acids

Lanolin fatty acids also, referred to as wool wax acids, include a variety of different acids. K. Motiuk, in the Journal Of The American Oil Chemists' Society, Vol. 56, No. 2, Pages 91 - 97 (1979), incorporated herein by reference, reviewed the analyses which have been performed on lanolin fatty acids and developed an average composition. The acids, their chain lengths and percent in the average composition are shown below.

| Acids | Chain length | % of lanolin fatty acids |
|---|---|---|
| Normal acids | $C_8-C_{38}$ | 10 |
| Iso acids | $C_8-C_{40}$ | 22 |
| Anteiso acids | $C_7-C_{41}$ | 28 |
| Normal α-hydroxy acids | $C_{10}-C_{32}$ | 17 |
| Iso α-hydroxy acids | $C_{12}-C_{34}$ | 9 |
| Anteiso α-hydroxy acids | $C_{11}-C_{33}$ | 3 |
| Normal ω-hydroxy acids | $C_{22}-C_{36}$ | 3 |
| Iso ω-hydroxy acids | $C_{22}-C_{36}$ | 0.5 |
| Anteiso ω-hydroxy acids | $C_{23}-C_{35}$ | 1 |
| Polyhydroxy acids | | 4.5 |
| Unsaturated acids | | 2 |
| Total | | 100% |

It should be recognized that the composition is an "average" composition and that the acids as well as the amount of any particular type of acid in a particular batch may vary somewhat from the average. It should also be recognized that commercial lanolin fatty acids may contain esters of the acids which are formed in the separation process, infra. The percentages for the lanolin fatty acid component of the present compositions include such esters.

Lanolin fatty acids can be separated from lanolin by saponification as described in Amerchol Lanolin Derivatives, Vol. II (1971), Amerchol, a unit of CPC International, Inc., incorporated herein by reference. Amerchol markets lanolin fatty acids under the names Amerlate LFA and Amerlate WFA. The lanolin fatty acids are present at a level of from about 5 to about 15%, preferably from about 8 to 12%, by weight of the moisturizing fraction.

Additional Oil Soluble Moisturizing Components

The skin conditioning emulsion compositions of the present invention may also contain emollient materials which help serve to moisturize the skin. Included in the list of acceptable ingredients are emollient/humectant agents such as hydrocarbon oils and waxes, monoglyceride esters, triglyceride esters, acetoglyceride esters, ethoxylated glycerides, alkyl esters, alkenyl esters, fatty alcohols, fatty acids other than the lanolin fatty acids, fatty alcohol ethers, fatty acid esters of ethoxylated fatty alcohols, lanolin alcohols, lanolin esters, hydroxylated lanolin derivatives, polyether derivatives, polyhydric alcohol esters, wax esters, beeswax derivatives, vegetable waxes, phospholipids and amides. Preferred types of emollient materials are fatty alcohols containing from about 12 to about 18 carbon atoms such as lauryl, myristyl, cetyl and stearyl and the comparable acids, and fatty acid esters of aliphatic alcohols where said esters contain from about 10 to about 31 carbon atoms such as ethyl laurate, isopropyl myristate, isopropyl palmitate, isopropyl behenate, isopropyl esters of lanolin fatty acids and hexadecyl acetate.


Emulsifier

An emulsifier is included as a part of the skin moisturizing fraction of the present compositions to emulsify the oil soluble components. Preferably, the emulsifier is present in an amount of from about 0.4% to about 68% by weight of the skin moisturizing fraction, more preferably from about 2 to about 22%. The emulsifier may be any agent which will form an emulsion with adequate stability. The proper emulsifier can be easily selected by the skilled person by utilizing the information he would have about the oily materials present in the particular composition selected.

The preferred emulsifiers useful in the present compositions are alkali metal or ethanolamine salts of fatty acids having from 12 to about 22 carbon atoms, alkali metal salts of sulfated fatty alcohols having from 12 to about 22 carbon atoms, glycerol esters, polyoxyethylene ethers and esters, polyethoxylated fatty acids, sugar esters, polyoxyethylene fatty ether phosphates, fatty acid amides, phospholipids, acyl lactylates and mixtures thereof. The most preferred emulsifiers include the alkali metal and ethanolamine soaps, the alkali metal salts of sulfated fatty alcohols, glycerol esters and polyoxethylene ethers and esters.

Examples of suitable emulsifiers include sodium stearate, potassium stearate, glycerol monostearate, sodium alkyl ($C_{12}$) ether sulfate, polyoxyethylene (8) stearate, polyoxyethylene (100) stearate, polyoxyethylene (20) sorbitan monolaurate, polyoxyethylene (2) oleyl ether, sucrose distearate, sucrose laurate, lauric. diethanolamide, lecithin, polyoxyethylene (10) oleyl ether phosphate and calcium stearoyl-2-lactylate.

Water

Distilled water is preferably used in the compositions of the present invention.

Water Phase Components

Many different water soluble materials may be present in the compositions of this invention. For example:

a) Additional humectants, such as glycerine, propylene glycol, alkoxylated glucose and hexanetriol at a level of from about 1% to about 20% of the total composition (the following percentages being on the same basis);

b) Thickening agents such as carboxyvinyl polymers, ethyl cellulose, polyvinyl alcohol, carboxymethyl cellulose, vegetable gums and clays such as Veegum ® (magnesium aluminum silicate, R. T. Vanderbilt, Inc.) at a level of from about 0.01% to about 6%;

c) Proteins and polypeptides at a level of from about 0.1% to about 3%;

d)  Preservatives such as the methyl, ethyl,
    propyl and butyl esters of hydroxybenzoic acid
    (Parabens - Mallinckrodt Chemical Corporation)
    EDTA and imidazolidinyl ureas (Germall 115 -Sutton
    Laboratories) at a level of from about 0.2% to
    about 2.5%; and

e)  An alkaline agent such as sodium hydroxide
    or an amine to neutralize, if desired, part of
    the fatty acids or thickener which may be present.

The present compositions may also contain agents
suitable for aesthetic purposes such as perfumes and
dyes.  These agents may be used in amounts sufficient
to achieve the desired fragrance and color (e.g. from
about 0.05% to about 1%).

The pH of the present compositions is preferably
in the range of about 4.5 - 9.0.

## Method of Manufacture

The compositions of the present invention generally have a lotion consistency and may be in the form of oil-in-water or water-in-oil emulsions with the former being preferred because of their more pleasing cosmetic properties.

The compositions of the present invention are preferably made by:

(A)   preparing the oil phase;

(B)   preparing the water phase; and

(C)   adding the oil phase to the water phase.

Step (A) is carried out by heating the oil phase materials to a temperature of about 75$^{O}$C. to about 100$^{O}$C.  Step (B) is carried out by heating the water phase materials to a temperature about the same as that of the oil phase.  The emulsion is formed by slowly adding the oil phase prepared in step (A) to the water phase prepared in step (B) with stirring.  Other ingredients may be added to the phase in which they are soluble prior to the mixing of the two phases or added directly to the mixed water and oil phases.

## Industrial Applicability

The compositions of the present invention are useful in the skin care field generally but particularly in the area of facial care.

The following examples further describe and demonstrate the preferred embodiments within the scope of the present invention.  Said examples are given solely for the purpose of illustration and are not to be construed as limitations of the present invention, as many variations thereof are possible without departing from the spirit and scope thereof.  Unless otherwise indicated, all percentages herein are by weight.

## EXAMPLE I

The following composition representative of the compositions of the present invention was prepared:

| Component | % |
|---|---|
| Isopropyl palmitate | 2.87 |
| Mineral Oil | 2.00 |
| Amerlate WFA® (lanolin fatty acids)[1] | 1.92 |
| OH Lan® (hydroxylated lanolin derivative high OH value)[1] | 1.29 |
| Stearic Acid | 1.00 |
| Cetyl Alcohol | 1.72 |
| Arlacel 165® (glycerol monostearate and polyoxyethylene stearate)[2] | 1.00 |
| Oleyl Oleate | 1.72 |
| Cholesterol | 1.00 |
| Carbopol 934® (polyvinyl polymer)[3] | 0.20 |
| Fructose | 3.60 |
| Triethanolamine | 0.40 |
| Methyl Paraben | 0.20 |
| Propyl Paraben | 0.10 |
| Titanium Dioxide | 0.20 |
| Perfume | 0.03 |
| Distilled Water | 80.75 |
| | 100.00% |

1. Supplied by Amerchol, a unit of CPC International, Inc.
2. Supplied by Atlas Chemicals, a division of ICI America, Inc.
3. Supplied by the B. F. Goodrich Company.

## EXAMPLE II

The following composition representative of the compositions of the present invention was prepared:

| Component | % |
|---|---|
| Isopropyl palmitate | 2.87 |
| Cetyl Alcohol | 5.44 |
| Stearic Acid | 1.00 |
| OH Lan® | 1.29 |
| Arlacel 165® | 1.00 |
| Amerlate WFA® | 1.92 |
| Cholesterol | 1.00 |
| Carbopol 934® | 0.20 |
| Sorbitol (70% aqueous solution) | 5.14 |
| Triethanolamine | 0.40 |
| Methyl Paraben | 0.20 |
| Propyl Paraben | 0.10 |
| Titanium Dioxide | 0.20 |
| Perfume | 0.03 |
| Distilled Water | 79.21 |
| | 100.00% |

## EXAMPLE III

The following composition representative of the compositions of the present invention was prepared:

| Component | % |
|---|---|
| Isopropyl Palmitate | 2.87 |
| Mineral Oil | 5.01 |
| Stearic Acid | 1.00 |
| Arlacel 165® | 1.00 |
| Oleyl Oleate | 1.72 |
| Amerlate WFA® | 1.92 |
| Cholesterol | 1.00 |
| Carbopol 934® | 0.20 |
| Sorbitol (70% aqueous solution) | 5.14 |
| Triethanolamine | 0.40 |
| Methyl Paraben | 0.20 |
| Propyl Paraben | 0.10 |
| Titanium Dioxide | 0.20 |
| Perfume | 0.03 |
| Distilled Water | 79.21 |
| | 100.00% |

All of the compositions delivered desirable skin conditioning while being easy to rub in and not leaving an undesirable greasy residue.

- 1 -

CLAIMS

1.    A skin conditioning composition characterized by:-

(A)    from 8 to 25% of skin moisturizing materials
       comprising:-
       (1)    an emulsifer;
       (2)    from 2.5 to 8% of a sterol or mixture
              of sterols;
       (3)    from 10 to 35% of a sugar or hydroxy
              alcohol selected from the group consisting
              of $C_5$ and $C_6$ monosaccharides, $C_5$ and $C_6$
              disaccharides, pentahydroxy alcohols,
              hexahydroxy alcohols and mixtures
              thereof; and
       (4)    from 5 to 15% of lanolin fatty acids; and
(B)    water.

2.    A skin conditioning composition according to Claim 1
characterized in that additional oil soluble, skin
moisturizing materials are present as a part of the 8 to
25% of skin moisturizing materials.

3.    A skin conditioning composition according to Claim 1
or 2 characterized in that the emulsifer is selected from
the group consisting of alkali metal soaps, ethanolamine
soaps, alkali metal salts of sulfated fatty alcohols
having from 12 to 22 carbon atoms, glycerol esters,
polyoxyethylene ethers and polyoxyethylene esters and
is present in an amount of from 0.4 to 68% of the amount
of skin moisturizing materials.

4.    A skin conditioning composition according to any of
Claims 1 to 3 characterized in the sterol is selected
from the group consisting of cholesterol, phytosterol, sitostearol, sitostearol pyroglutomate, 7-dehydrocholesterol lanosterol, and mixtures thereof.

5. A skin conditioning composition according to any of Claims 1 to 4 characterized in that the sugar or hydroxy alcohol is selected from the group consisting of arabinose, xylose, ribose, glucose, galactose, mannose, fructose, lactose, maltose, gentiobiose, celliobiose, sucrose, arabitol, xylitol, adonitol, mannitol, sorbitol, dulcitol, and mixtures thereof.

6. A skin conditioning composition according to any of Claims 1 to 5 characterized in that the sterol is cholesterol.

7. A skin conditioning composition according to any of Claims 1 to 6 characterized in that the sugar or hydroxy alcohol is sorbitol.

8. A skin conditioning composition according to any of Claims 1 to 7 characterized in that in addition it contains from 0.01 to 6% of a thickening agent.

9. A skin conditioning composition according to any of Claims 1 to 8 characterized in that the amount of emulsifier is from 0.25 to 2.5%.

10. A skin conditioning composition according to any of Claims 1 to 9 characterized in that the amount of skin moisturizing materials is from 12 to 15% of which 2.5 to 4% is cholesterol, 10 to 25% is sorbitol and 8 to 12% is lanolin fatty acids.